Europäisches Patentamt

European Patent Office

Office européen des brevets

(19)

(11)  EP 1 371 974 A1

(12)  **EUROPEAN PATENT APPLICATION**
published in accordance with Art. 158(3) EPC

(43) Date of publication:
**17.12.2003  Bulletin 2003/51**

(51) Int Cl.⁷: **G01N 27/26**, G01N 27/327,
G01N 27/416, G01N 33/53

(21) Application number: **02712364.5**

(22) Date of filing: **14.02.2002**

(86) International application number:
**PCT/JP02/01269**

(87) International publication number:
**WO 02/065112 (22.08.2002 Gazette 2002/34)**

(84) Designated Contracting States:
**AT BE CH CY DE DK ES FI FR GB GR IE IT LI LU
MC NL PT SE TR**

(30) Priority:  **14.02.2001  JP  2001037141**

(71) Applicants:
• **Tanioka, Akihiko
  Ohta-ku, Tokyo 145-0061 (JP)**
• **RIKEN
  Wako-shi, Saitama 351-0198 (JP)**
• **S.T. Research Co., Ltd
  Tokyo 150-0012 (JP)**

(72) Inventors:
• **TANIOKA, Akihiko
  Ohta-ku, Tokyo 145-0061 (JP)**
• **YAMAGATA, Yutaka, c/o RIKEN
  Wako-shi, Saitama 351-0198 (JP)**
• **INOUE, Kozo, c/o S T RESEARCH CO. LTD
  Tokyo 150-0012 (JP)**

(74) Representative: **Fenlon, Christine Lesley
  Haseltine Lake & Co.,
  Imperial House,
  15-19 Kingsway
  London WC2B 6UD (GB)**

(54)  **APPARATUS FOR DETECTING INTERACTION BETWEEN BIOPOLYMER AND LIGAND AND METHOD THEREOF**

(57)    A apparatus is provided for detecting interactions between biomolecules and ligands, the apparatus comprises: a reaction block constituting a reaction system comprising a reaction region including immobilized biomolecules, a supply flow channel connected to the reaction region for supplying a sample solution, and a recovery flow channel connected to the reaction region for recovering the sample solution which passes through at least a part of the reaction region; and a measuring block for measuring streaming potential of the immobilized biomolecules while the sample solution is supplied to the reaction region from the supply flow channel and the sample solution passing through at least a part of the reaction region is collected by the recovery flow channel. Thereby it is possible to detect interactions between a number of biomolecules and ligands at one time.

*FIG. 1*

EP 1 371 974 A1

## Description

Background of the Invention

Field of the Invention

The present invention relates to an apparatus and a method for detecting interactions between biomolecules and ligands.

Related Art Statements

Reactions for the most part within the living body begin with bonding or binding of biomolecules, especially proteins, to compounds (which are referred to as a "ligand") which preferentially combine with specific proteins.

Genome sequences of human beings have been determined, then, it has been required to clarify functions of individual genes, i.e. functions of proteins derived from the individual genes. Accordingly, the techniques for detecting interactions between proteins and ligands are increasingly important in these days. Because preferences or specificities of combinations of proteins and ligands are very high, the detecting techniques have been utilized as analytical techniques in many fields such as clinical diagnosis or environmental issues. Thus, many kinds of techniques for detecting combinations of proteins and ligands have been developed.

For instance, label immunoassay methods such as Radio Immunoassays, Enzyme Immunoassays (EIA/ELISA), and Fluorescence Immunoassays (FIA) using known ligands labeled with radioactive isotopes, enzymes, and fluorescent compounds are well known (refer to a Japanese document "Analytical Chemistry (Bunseki)", pp. 839-843, 1999). Although these methods may be classified into several groups by techniques for separating antibodies and antigens, a basic principle of the methods is to label various known antibodies or antigens with radioactive isotopes, enzymes, or fluorescent compounds to detect combinations with targeted antibodies or antigens. In particular, enzyme immunoassays (EIA/ELISA) and fluorescence immunoassays (FIA) are most often used in these days.

A technique using a SPR sensor based on Surface Plasmon Resonance (SPR) phenomenon has recently been developed (refer to a Japanese document " Analytical chemistry (Bunseki)", 1997, pp. 362-368). This technique is based upon a phenomenon that when a concentration is changed in a vicinity of a thin metal film formed on a substrate, the concentration shift affects on a refraction index to shift an incident angle which decreases an intensity of reflected light. In practice, after one of a pair of compounds to be detected is fixed on a substrate using a dextran, a sample solution containing the other of the compounds is poured on metal film and a change of the incident angle is measured to detect interactions or bindings between the pair of compounds.

A mechanochemical method has been also developed, in which biomolecules are regarded as an elastic body and bindings are detected by measuring a change of tension due to a shift of a higher order structure caused by bonds of ligands with biomolecules (refer to a document "Anal. Biochem. 201", 1992, pp. 68-79).

At a direct interface between a solid phase and a solution phase, electrolytes in a solution are absorbed on a surface of a solid. Accordingly positive and negative charges are separated at the interface and a surface polarization is occurred to generate an electric double layer at the interface. Because streaming potential or zeta potential derived from the electric double layer represents a condition of the surface of the solid, the streaming or zeta potential is utilized for various purposes in many sorts of fields using polymer membranes, fibers and a variety of particles. It is known that this streaming potential or zeta potential is changed by bonding of biomolecules with ligands (refer to a document "Biosensors 2", 1986, pp. 89-100).

In order to detect an interaction between proteins and ligands, various techniques have been developed as described above. However, in order to investigate proteins whose functions are unknown and to utilize investigation results for various purposes, it has been desired to develop a technology for detecting the bonding of a great number of proteins with ligands at one time.

In this case, regardless of proteins whose functions are known or whose ligands are known, it is preferable that detecting techniques do not need to label ligands with enzymes or fluorescence compounds, because it is often the case that: ligands are hard to be obtained; labeling compounds are hard to be bonded; or activities of functions are degraded by labels. Taking account of these facts, above described techniques and methods have many problems.

The label immunoassay method such as enzyme immunoassay (EIA/ELISA) and fluorescence immunoassay (FIA) need known ligands or labeling compounds, and therefore this method is not applicable to proteins whose functions are unknown.

Although the SPR sensor does not require known ligands or labeling compounds, nowadays this sensor is mainly used for detecting bonds between comparatively large molecules. It can be said that the sensor cannot detect bonds between molecules whose molecular weight is less than several hundred Daltons even though most appropriate condition is kept. This limit would be a very big restriction when the sensor is applied to proteins whose functions are unknown.

The mechanochemical method has following major advantages: the method does not need known ligands or labeling compounds like as the SPR sensor; and the method can be applied to low molecular weight compounds (there are some reports that the method can detect bonding of proteins with Ca or Mg). However, in order to detect simultaneously various bonds of a number of proteins with ligands using the mechanochemical method, a detecting apparatus might be very large and more complex.

Trials for applying the streaming potential or zeta potential to the detection of biomolecules as well as compounds which are combined with the biomolecules have been performed since the 1970s. Glad et al. reported that bonds of IgG with Protein A could be measured with the streaming potential (refer to a document "Biosensors 2" 1986, pp. 89-100). Nishizaki reported that bonds of human serum albumin (HSA) with anti HSA antibodies were measured quantitatively by a change of the zeta potential (refer to a patent publication: Japanese Patent Laid Open No. 6-265,551). Koch et al. investigated experimentally and theoretically a relationship between bonds of proteins and streaming potential using lysozyme (refer to a document "Biosensors & Bioelectronics 14", 1999, pp. 413-421).

As described above, the streaming potential or zeta potential has been investigated to develop a method applicable to the detection of bindings between biomolecules and ligands without using known ligands or known labeling compounds and regardless of molecular mass. However, the methods have a problem in terms of immobilization of biomolecules. In respect of the immobilization: Glad et al. immobilized biomolecules in a tube packed with porous material; Nishizaki immobilized them onto latex particles; and Koch et al. fixed in a capillary packed with fused-silica. Thus conventional methods above described could not detect interactions between a number of biomolecules and ligands at one time.

On the other hand, an electrospray deposition method, which is a technique for forming a minute film or spots of biomolecules is disclosed (refer to documents "Anal. Chem. 71" 1999, pp. 1415-1420, "Anal. Chem. 71" 1999, pp. 3110-3117"). For example, it is assumed that a protein microchip including a number of protein spots is manufactured using this method. Interactions between each protein of respective spots with compounds contained in a sample solution may be detected by measuring streaming or zeta potential when the sample solution is fed onto the chip.

Objects of the Invention

It is an object of the present invention to provide an apparatus and a method for detecting combinations or bindings of macromolecules or biomacromolecules with compounds which preferentially associate with specific macromolecules without the need for a known ligand or a known labeling compound. It is another object of the present invention to provide an apparatus and a method for detecting combinations, at one time, of a number of biomolecules with compounds which preferentially associate with specific biomolecules without the need for a known ligand or a known labeling compound.

Summary of the Invention

According to the invention, an apparatus for detecting interactions between biomolecules and ligands comprises:

a reaction block constituting a reaction system comprising a reaction region including immobilized biomolecules, a supply flow channel connected to the reaction region for supplying a sample solution, and a recovery flow channel connected to the reaction region for recovering the sample solution which passes through at least a part of the reaction region; and

a measuring block for measuring streaming potential of the immobilized biomolecules while the sample solution is supplied to the reaction region from the supply flow channel and the sample solution passing through at least a part of the reaction region is collected by the recovery flow channel.

In a preferable embodiment of the apparatus according to the present invention, the apparatus is characterized in that:

said reaction block is formed by a first and a second substrates which are bonded mutually such that the reaction region, the supply flow channel and the recovery flow channel are formed at a boundary, more properly a gap, between flat surfaces of the first and second substrates; and

said reaction block further comprises an inlet and an outlet for communicating the supply flow channel and recovery flow channel to outside.

In another embodiment of the apparatus according to the present invention, the apparatus is characterized in that said biomolecules are immobilized using an electrospray deposition method.

In still another embodiment of the apparatus according to the present invention, the apparatus comprises:

a pair of minute electrodes which are disposed respectively on a side of the supply flow channel and on a side of the recovery flow channel of the reaction region having the biomolecules immobilized thereto; and

a measuring circuit for measuring a streaming potential generated between the pair of electrodes.

In still another embodiment of the apparatus according to the present invention, the apparatus is characterized in that said pair of electrodes is ISFETs.

In still another embodiment of the apparatus according to the present invention, the apparatus comprises:

an array of a plurality of films or spots of the immobilized biomolecules;
plural pairs of electrodes, electrodes of each pair of which are disposed on a side of the supply flow channel and recovery flow channel, respectively; and
a measuring circuit for measuring streaming potentials generated between the plurality of pairs of electrodes provided for respective films or spots.

In still another embodiment of the apparatus according to the present invention, the apparatus comprises:

a pair of pressure detectors which are disposed respectively at the inlet and the outlet; and
a calculating circuit for calculating a zeta potential based upon the measured streaming potential and the detected pressure.

According to another aspect of the present invention, a method for detecting interactions between biomolecules and ligands using a reaction block constituting a reaction system comprising a reaction region including immobilized biomolecules, a supply flow channel connected to the reaction region for supplying a sample solution, and a recovery flow channel connected to the reaction region for recovering the sample solution which passing through at least a part of the reaction region, comprises the step of measuring a streaming potential of the immobilized biomolecules while supplying the sample solution to the reaction region from the supply flow channel and recovering the sample solution passing through at least a part of the reaction region from the recovery flow channel.

In a preferable embodiment of the method according to the present invention, the method comprises the steps of:

detecting pressure of the sample solution at an inlet on a side of the supply flow channel and an outlet on a side of the recovery flow channel, respectively; and
calculating zeta potential based upon the measured streaming potential and the detected pressure.

In an explanation described above, the present invention has been mainly explained as the apparatuses, however the present invention may include methods corresponding to the apparatuses.

Now a principle of the apparatus and method for detecting interactions between biomolecules and ligands according the present invention will be described in detail.

At an interface contacting directly a solid phase with a liquid phase, electrolytes in solution are absorbed on a surface of a solid. Accordingly a separation of positive and negative charges, i.e. surface polarization occurs at the interface to generate an electric double layer at the interface. Under an existence of the electric double layer at the interface, when the solid phase and liquid phase are relatively shifted, an electric field is induced. This phenomenon is referred to as an "electrokinetic phenomenon at interface", this phenomenon is remarkable when using: a porous membrane including a great number of capillary vessels; a capillary; or a system consisting of a solution and two flat plates which are mutually separated with interposing a narrow space therebetween. At a contact surface between a solid phase and a liquid phase, when the solid phase and liquid phase are relatively moved, a thin layer of the liquid phase is stuck on the contact surface of the solid phase and is moved together with the solid phase. An interface between this fixed thin liquid phase layer and the moving liquid phase is referred to as a "slip surface". A potential difference between the slip phase and a bulk liquid is referred as to "zeta ($\zeta$ potential" or "electrokinetic potential". When an electrolyte solution flows through a porous membrane, a capillary, or a space between two flat plates by applying an arbitrary pressure difference ($\Delta P$), a potential difference appears between both boundary faces of the membrane, or between both ends of the capillary, or between both ends of the plates. This potential difference is referred to as "streaming potential".

There is a following relation between the streaming potential ($\Delta E$) and the zeta potential ($\zeta$).

$$\frac{\Delta E}{\Delta P} = \frac{\varepsilon \zeta}{\eta \lambda} \qquad (1)$$

where $\Delta P$ denotes the pressure difference of the solution between both boundary faces of the membrane, or between both ends of the capillary or the plates, $\varepsilon$ signifies a dielectric constant of the solution, $\eta$ represents a viscosity of the solution, and $\lambda$ represents a conductivity of the solution. As can be understood from the equation (1), $\varepsilon$, $\eta$ and $\lambda$ are values inherent to the solution, which values may be known from documents or by measuring the solution. Accordingly, when $\Delta E$ is measured while changing $\Delta P$, $\xi$ can be obtained. $\zeta$ can be contemplated as a parameter representing features or functions of the surface of the membranes, capillary, or flat plates.

$\Delta E$ is generally measured at between both boundary faces of the membrane, or between both ends of the capillary, or between both ends of the plates. When the measurement is done at two halfway points between these end points of a fine porous of the membrane or the capillary or the plates, at which pressure difference is to be $\Delta P_i$, a local potential difference $\Delta E_i$ between the two halfway points can be obtained. Accordingly an apparent local zeta potential $\zeta_i$ between the two points is obtained. Here, if conditions of $\varepsilon$, $\eta$ and $\lambda$,

etc. can be kept constant, the measured streaming potential instead of the zeta potential may be treated as a measure of an evaluation of a surface(s). Thus, when n regions (which are referred to as "local surfaces")=having different characteristics are provided on a surface(s) of a fine porous of the membrane, or the capillary, or flat plates, and n pairs of electrodes are disposed at both ends of respective local surfaces, n streaming potentials can be obtained. Here, n local pressure differences between the n pairs of electrodes on the n local surfaces can be obtained from a measurement of both total pressure difference $\Delta P$ and positions of the pairs of electrodes. Finally, apparent local zeta potentials can be calculated.

Since the streaming potential and zeta potential are changed in consequence of bonds of biomolecules with ligands, interactions between biomolecules and ligands can be detected without using a known ligand or a known labeling compound by measuring the streaming potential and zeta potential change.

Brief Description of the Drawings

Fig. 1 is a cross-sectional view showing a basic structure of a detecting apparatus 10 according to the invention;

Fig. 2 is a schematic diagram illustrating a first substrate 21 of the detecting apparatus according to the invention;

Fig. 3 is a schematic diagram representing a second substrate 34 of the detecting apparatus according to the invention;

Fig. 4 is a cross-sectional view of a detecting apparatus 50 of double-sided type according to the invention;

Fig. 5 is a cross-sectional view of a substrate having pin type electrodes;

Fig. 6 is a cross-sectional view of a substrate having land electrodes;

Fig. 7 is a schematic diagram showing electrodes using IS-FETs (Ion Sensitive Field Effect Transistors);

Fig. 8 is a cross-sectional view of a substrate comprising IS-FET type electrodes;

Fig. 9 is a schematic diagram illustrating an array-type detecting apparatus in which a number of biomolecules spots and a number of electrodes are arranged in form of an array;

Fig. 10 is a graph showing relationship between potential difference and solution concentration, which are obtained when a $CaCl_2$ solution (HEPES buffer, at pH 7.5) is fed at flow rate of 1 ml/minute; and

Fig. 11 is a graph showing difference of a streaming potential, which is measured with a substrate having spots i.e. biomolecules immobilized thereto, and a streaming potential, which is measured with a substrate without the spots.

Detailed Description of the Preferred Embodiments

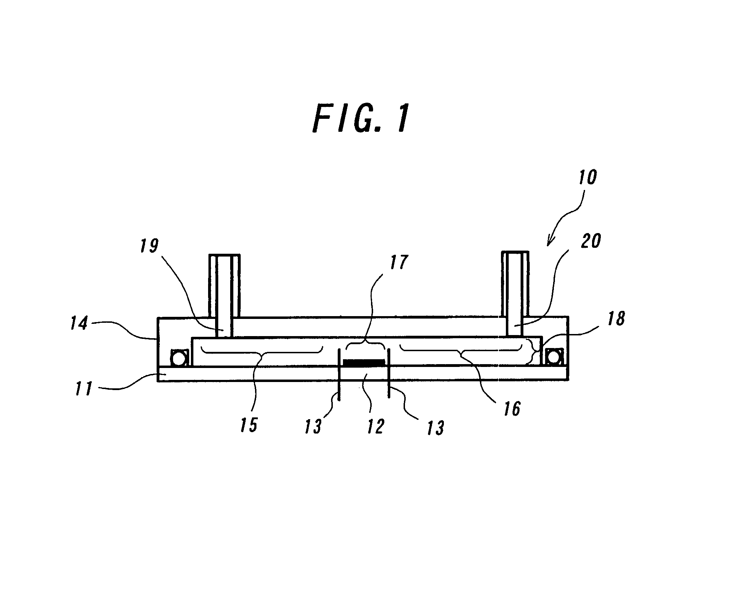

Several preferred embodiments of the apparatus according to the present invention will be described with reference to the accompanying drawings. Fig. 1 is a cross-sectional view showing a basic structure of a detecting apparatus 10 according to the invention. The detecting apparatus 10 comprises a reaction block constituting a reaction system which reaction block is formed by bonding a flat face of a first substrate 11 with a flat face of second substrate 14, and a supply flow channel(s), a recovery flow channel(s), and a reaction region(s), i.e. a site(s) are formed at the contact surface of the substrates. A sample solution containing biomolecules has been immobilized onto the surface of the substrate 11 into a spot(s) 12 or a film(s) of the biomolecules. A pair of electrodes 13 is located respectively upstream and downstream of the spot 12. The second substrate 14 has a concave portion 18 which form, after bonding the first substrate with the second substrate, a supply flow channel 15, a recovery flow channel 16, and a reaction region 17. Both ends of the concave potion 18 have an inlet 19 and an outlet 20, respectively, for supplying and recovering a sample solution to communicate the both flow channels with outside. The inlet 19 and the outlet 20 are connected to a supply tube and a recovery tube, respectively. In order to prevent liquid leak, a groove potion in form of ring, which is for engaging an O-ring, is formed at the edge of the contact surface of one or both of the substrates.

The first substrate 11 is made of acrylic and is about $75\times25$ mm in area and about 1 mm in thickness. Also, the second substrate 14, which acts as a flow path structure, is made of acrylic and is approximately $75\times25$ mm in area and about 5 mm in thickness. As shown in the figure, two rod-like platinum electrodes 13 are disposed in front and rear (at upstream side and downstream side) of a macromolecules film on the first substrate 11, respectively, which electrodes have a diameter of 0.2 mm and is protruded approximately 0.5 mm from the top surface of the substrate. The first and second flow channels are sealed by the oval O-ring, which flow channels are approximately 35 mm in length, 5 mm in width, and 1 mm in thickness. A solution is injected into the inlet from a supply tube by pump such as a peristaltic pump. A biomolecules spot(s) 12 is formed using the electrospray deposition method and the spot is cross-linked by glutaraldehyde.

At the time of measurement, a sample solution containing ligands is injected via the supply tube into the inlet by applying pressure. Then the sample solution is fed to the reaction region, in which measurement is to be performed therein, local streaming potential generated between the pair of electrodes 13 is measured in the circumstance. Bindings between substances and ligands existing on a local surface are determined based upon a change of the measured streaming potentials or a difference between the measured streaming potential and a reference potential with a substrate without biomolecules in this manner.

In Fig. 1, the apparatus comprising single measuring unit including single biomolecules firm and the pair of electrodes is explained by an example, the detecting apparatus may be configured such that the apparatus comprises n measuring unit in form of an array (n spots with n pairs of electrodes located in front and rear of the each spot), it is possible to measure streaming potentials with bonds between two or more kinds of biomolecules and ligands at one time using this apparatus. For instance, "n" is preferably set to a range of 1-10000.

For example, as a modification of the embodiment according to the present invention, the detecting apparatus may be configured as following:

(1) A supply flow channel(s) and a recovery flow channel(s) and a reaction region(s), which are formed at a joint surface between a first and a second substrates, which have a width equal to or more than 50 nm and less or equal to 1 cm.

(2) The first and second substrates have an area equal to or more than 1 mm$^2$ and less or equal to 900 cm$^2$.

(3) The first and second substrates are made of metal material, inorganic material, or organic material, which all material having conducting properties.

(4) Electrodes are platinum, platinum black, or silver/silver chloride, if the electrodes are in form of linear the electrodes have a diameter equal to or more than 0.05 mm and less or equal to 2 mm, if the electrodes are in form of a flat plate the electrodes equal to or more than 0.05 mm and less or equal to 2 mm in diameter and equal to or more than 0.1 mm and less or equal to 1 cm in width.

(5) An electrolyte solution to be used is a solution containing univalent electrolyte such as NaCl, or bivalent electrolyte such as CaCl$_2$, or other multivalent electrolyte, its concentration is in a range of 10$^{-5}$ mol/1 to 5 mol/l.

(6) A pressure difference $\Delta P$ between an end of the supply flow channel and an end of the recovery flow channel is in a range of 0.01 to 1 atmosphere.

(7) Minute films or spots are equal to or more than 1 $\mu$m$^2$ and less or equal to 1 cm$^2$ in area.

(8) A spacing between the electrodes (i.e. pitch of electrodes) is equal to or more than 1 $\mu$m and less or equal to 1 cm.

(9) Measuring circuits to be used in this apparatus may measure a potential difference between the electrodes in an order of 1 $\mu$V to 1000 mV.

(10) Each of the measuring circuits for measuring potential comprises a pair of electrodes, which are disposed respectively at regions without films or spots and in front of and rear (upstream side and downstream side) of the minute biomolecules film or spot, in which proteins or DNAs are deposited thereon by the electrospray method.

While supplying the sample solution, the potential differences between the electrodes are measured. In this manner, by finding a difference between the measured potential difference and potential previously measured in condition without the immobilized biomolecules, bindings between biomolecules and ligands are detected.

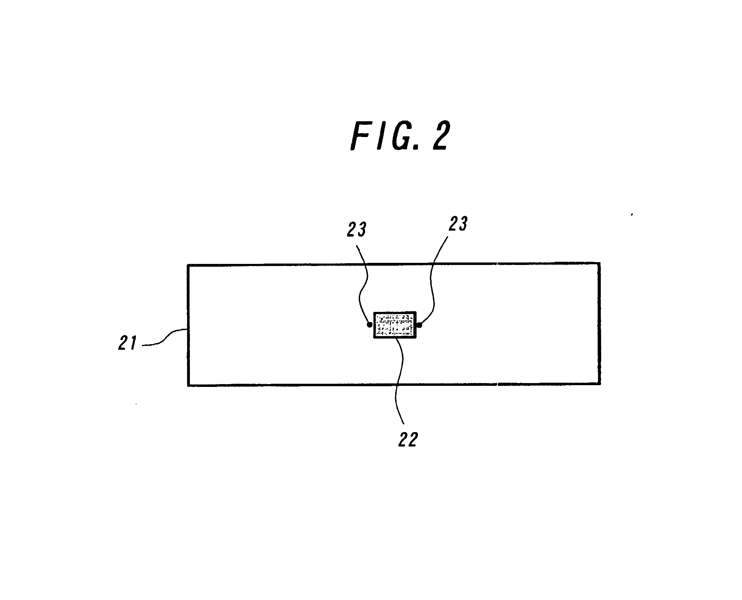

Fig. 2 is a schematic diagram illustrating a first substrate 21 of the detecting apparatus according to the invention. A biomolecules spot (film) 22 is immobilized on flat surface of the first substrate 21. A pair of electrodes 23 are disposed at upstream side (i.e. on a side of an inlet) and downstream side (i.e. on a side of outlet) of the spot 22, respectively.

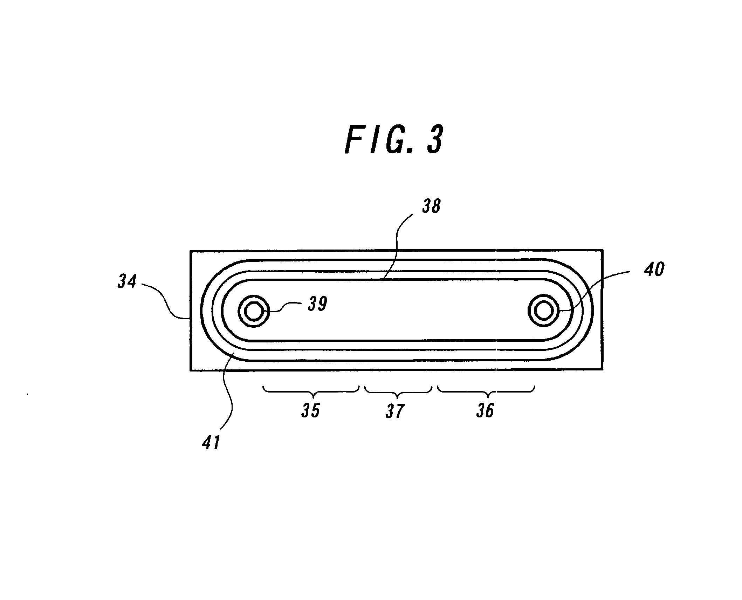

Fig. 3 is a schematic diagram representing a second substrate 34 of the detecting apparatus according to the invention. The second substrate 34 has a concave portion 38 which form a supply flow channel 35, a recovery flow channel 36, and a reaction region 37 at a gap between the both substrate after bonding the two substrates. Both ends of the concave potion 38 have an inlet 39 and an outlet 40, respectively, for recovering a sample solution, and to communicate the both flow channels to outside. In order to prevent liquid leak, the substrate 34 has a groove potion 41 for containing an O-ring for sealing.

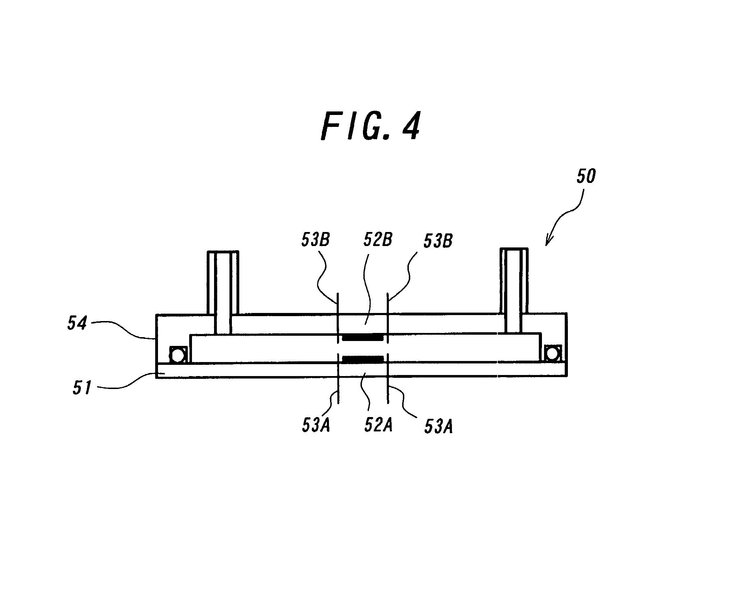

Fig. 4 is a cross-sectional view of a detecting apparatus 50 of double-sided type according to the invention. This double-sided type detecting apparatus 50 comprises a reaction block including a reaction system which is configured such that flat surfaces of a first substrate 51 and a second substrate 54 are bonded to form supply and recovery flow channels and a reaction region in a gap therebetween. Biomolecules spot 52A is immobilized on a surface of the first substrate 51. A pair of electrodes 53A are disposed at upstream side and downstream side of this spot 52A, respectively. Also, Biomolecules spot 52B is immobilized on a surface of the second substrate 54. A pair of electrodes 53B is disposed at upstream side and downstream side of this spot 52B. In this manner, in the double-sided type, the biomolecules spot 52B and the pair of the electrodes 53B are disposed on the upper substrate 54 as well as the single-sided type mentioned above, so that streaming potentials can be detected in higher sensitivity.

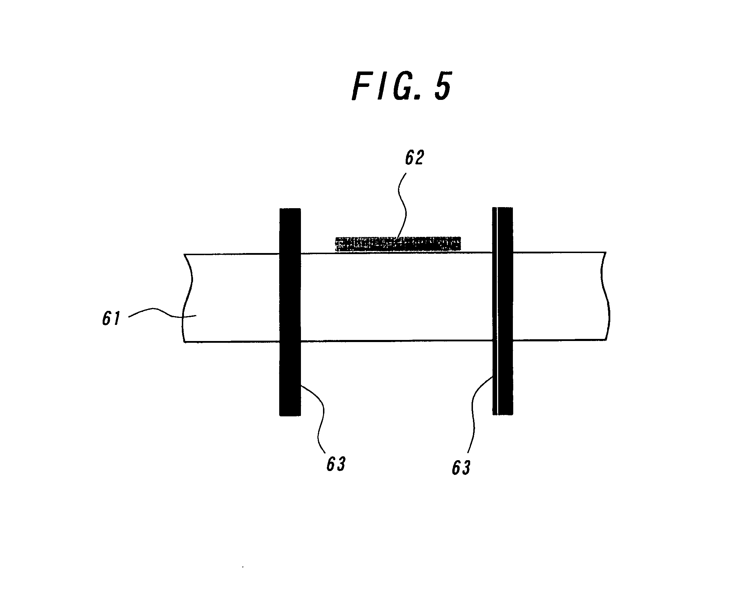

Fig. 5 is a cross-sectional view of a substrate comprising pin type electrodes. In this embodiment, a biomolecules spot 62 is immobilized on a surface of a substrate 61. A pair of pin-type electrodes 63 are disposed respectively at upstream side and downstream side of the spot. The substrate 61 is penetrated by these electrodes from the bottom to top surface of the substrate 61, and to expose heads of the electrodes in a reaction region.

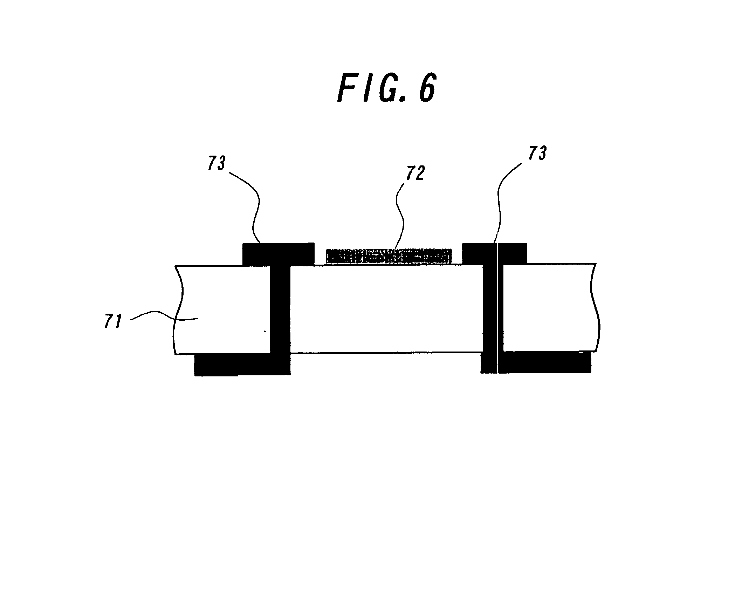

Fig. 6 is a cross-sectional view of a substrate comprising land type electrodes. In this embodiment, a biomolecules spot 72 is immobilized on a surface of a substrate 71. A pair of land electrodes 73 are disposed at upstream side and downstream side of the spot, respectively. These land electrodes 73 are connected to wires which pierce through form the bottom to top surface of the substrate 71.

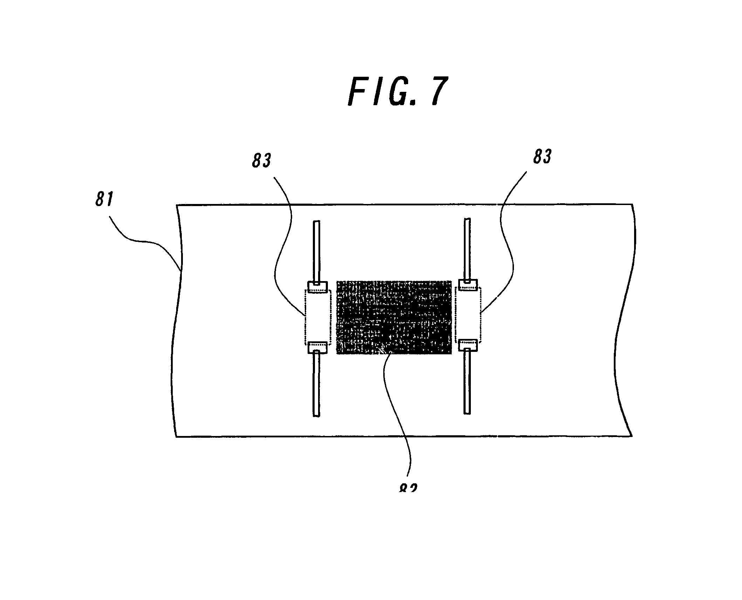

Fig. 7 is a schematic diagram showing electrodes using IS-FETs (Ion Sensitive Field Effect Transistors). In this embodiment, a biomolecules spot 82 is immobilized on a surface of a substrate 81. A pair of IS-FET type electrodes 83 is disposed at upstream side and downstream side of the spot.

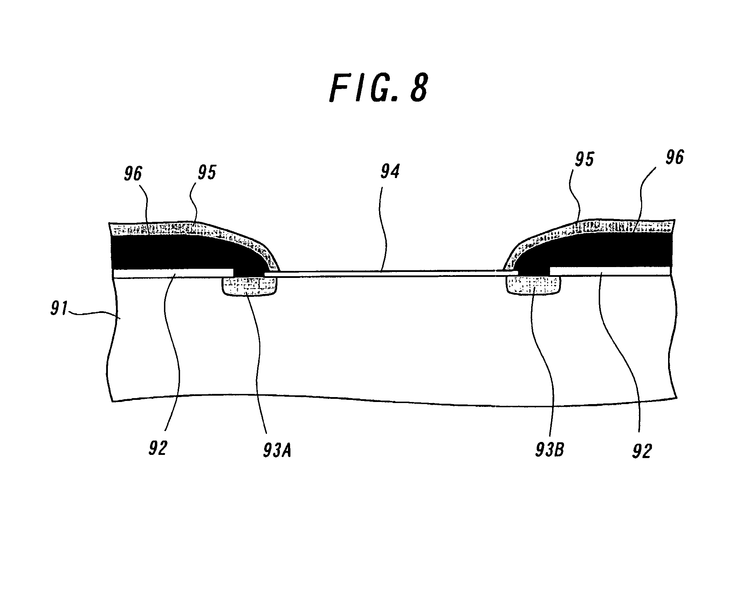

Fig. 8 is a cross-sectional view of a substrate comprising IS-FET type electrodes. As shown in the figure, reference numbers 91, 92, 93A, 93B, 94, 95, and 96 represent a substrate, an oxide film, a drain, a source, a gate oxide film, a protection layer, and an electrode wiring, respectively. When the IS-FET type electrodes as shown are used, since the electrodes are very sensitive to a potential change on the top surface of the gate oxide layer, so that streaming potential can be detected in higher sensitivity.

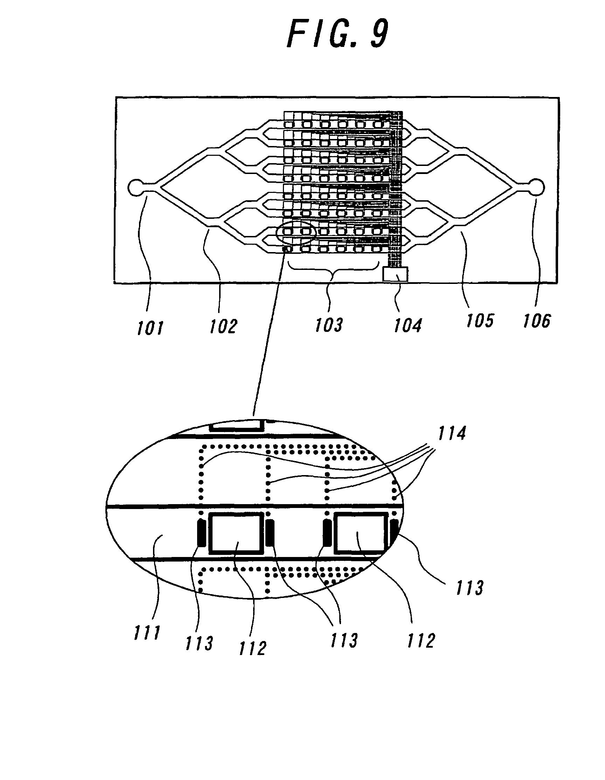

Fig. 9 is a schematic diagram illustrating an array-type detecting apparatus in which a number of biomolecules spots and a number of electrodes are arranged in form of an array. As shown in the upper part of Fig. 9, reference numbers 101, 102, 103, 104, 105, and 106 represent an inlet, a distribution circuit (channels), reaction regions (i.e. regions which are to be measured), a connector for connecting electrodes wiring, a recovery flow circuit (channels), and an outlet, respectively. As shown in the lower part of Fig. 9, the lower part is an enlarged view of a part of the reaction regions 103, reference numbers 111, 112, 113, and 114 represent minute flow channels, biomolecules spots, a many number of pairs of electrodes, and a wiring, respectively. Although as shown each of spots 112 is substantially same to that of a basic arrangement, each of the electrode wiring is collected to one point with aid of a pattern provided on a backside of the substrate and potential change on the each electrodes can be quickly detected. Each of the spots 112 is configured such that a sample solution is uniformly and continuously fed to the each spot with aid of minute flow channels 111. According to this embodiment, by forming spots of many different kinds of biomolecules on the substrate, it is possible to configure a detecting system which can simultaneously detect bonds of many different kinds of substances.

As described above by utilizing a print-circuit technology, the electrodes can be electrodes in form of different structure such as a land type. When electrodes are provided in form of an array, electrode wiring is connected to outside lines of the substrate via the front or rear surface of the substrate. An amplifier may also be disposed beneath of the electrodes.

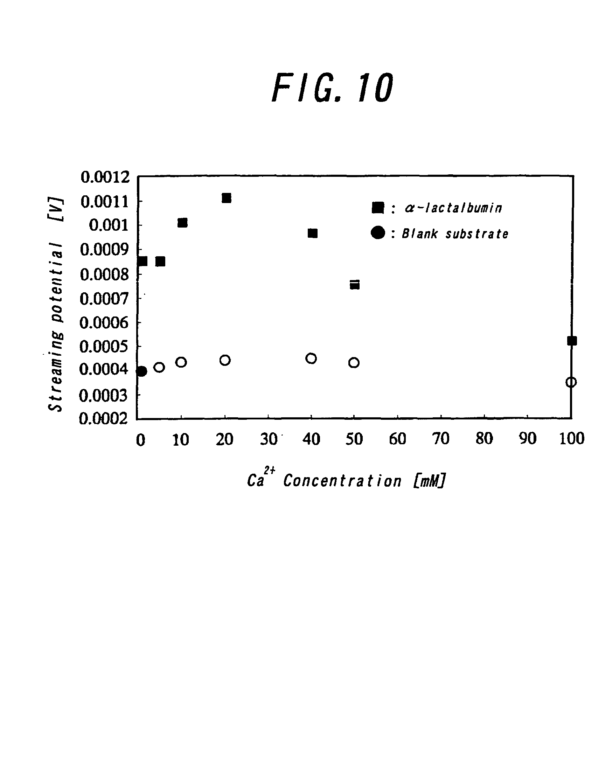

We actually performed an experiment to measure bonds or binding between biomolecules and ligands using the detecting apparatus according to the present invention. α-lactalbumin was deposited onto a substrate of 5×5 mm using the electrospray method, and to configure detecting apparatus according to the present invention comprising a block which include the substrate. Fig. 10 is a graph showing relationship between potential difference and solution concentration, which is obtained when a CaCl$_2$ solution (HEPES buffer, at pH 7.5) is fed at flow rate of 1 ml/minute in this detecting apparatus. The detecting apparatus to be used was a basic type arrangement which is shown in Fig. 1. A blank test was performed on the same condition using the substrate without the deposited α-lactalbumin while pouring the CaCl$_2$ solution. The substrate is made of acryl. A region, which is to be deposited the α-lactalbumin, of a surface of the substrate was coated with Pt-Pd alloy by a sputter deposition process. As shown in the graph, it is obvious that there is a difference between streaming potential, of the substrate with the spots, and that of the substrate without the spots. Thus, the graph represents that the detecting apparatus or the method according to the invention can measure streaming potential to characterize various types of proteins or DNAs.

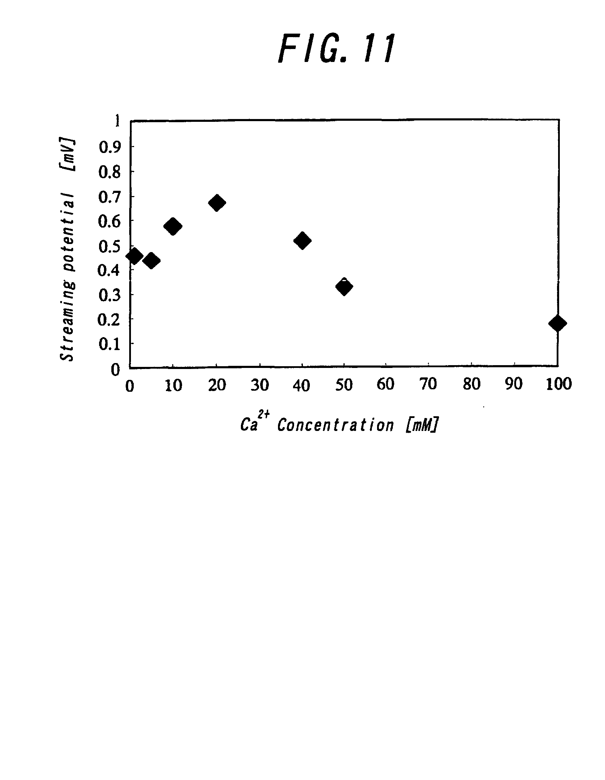

Fig. 11 is a graph showing difference between a streaming potential, which is measured with a substrate having biomolecules or spots immobilized thereto, and a streaming potential, which is measured with a blank substrate, i.e. without the spots.

While the present invention has been described with respect to a several embodiments and these embodiments are exemplary. It should be understood that numerous modifications and variations could be made therefrom. It is intended that the appended claims cover all such modifications and variations as fall within the true spirit and scope of this present invention. For example, although not mentioned in the embodiments, pressure gauges (detectors) may be provided at upstream side and at downstream side respectively in the detecting apparatus, in addition to the electrodes for measuring potential difference. Thus pressure difference between the gauges can be obtained. When the detecting apparatus comprises a computing unit such as CPU, zeta potential may be calculated form measured streaming potential. Those skilled in the art can easily evaluate bonds or binding between biomolecules and ligands based upon the measured streaming potential or the calculated zeta potential.

Industrial applicability

According to the apparatuses or the methods for detecting interactions between biomolecules and ligands of the present invention, for example, it is possible to detect bonds or bindings between various types of proteins and compounds, which preferentially combines with specific proteins without the need for a known ligand or a known labeling compound. That is the present apparatus or the method can easily and simply detect interactions between them. Also, it is expected that the apparatuses or the methods of the present invention are applied for various fields such as clinical diagnosis or environmental analysis.

## Claims

1. A apparatus for detecting interactions between biomolecules and ligands, comprising:

a reaction block constituting a reaction system comprising a reaction region including immobilized biomolecules, a supply flow channel connected to the reaction region for supplying a sample solution, and a recovery flow channel connected to the reaction region for recovering the sample solution which passes through at least a part of the reaction region; and a measuring block for measuring streaming potential of the immobilized biomolecules while the sample solution is supplied to the reaction region from the supply flow channel and the sample solution passing through at least a part of the reaction region is collected by the recovery flow channel.

2. The apparatus according to claim 1, wherein said reaction block is formed by a first and a second substrates which are bonded mutually such that the reaction region, the supply flow channel and the recovery flow channel are formed at a boundary between flat surfaces of the first and second substrates, and wherein said reaction block further comprises an inlet and an outlet for communicating the supply flow channel and recovery flow channel to outside.

3. The apparatus according to claim 1, wherein said biomolecules are immobilized using an electrospray deposition method.

4. The apparatus according to claim 2, wherein said biomolecules are immobilized using an electrospray deposition method.

5. The apparatus according to claim 1, comprising:

a pair of minute electrodes which are disposed respectively on a side of the supply flow channel and on a side of the recovery flow channel of the reaction region having the biomolecules immobilized thereto; and measuring circuit for measuring a streaming potential generated between the pair of electrodes.

6. The apparatus according to claim 1, wherein said pair of electrodes is ISFETs.

7. The apparatus according to claim 1, comprising:

an array of a plurality of films or spots of the immobilized biomolecules; plural pairs of electrodes, electrodes of each pair of which are disposed on a side of the supply flow channel and the recovery flow channel, respectively; and measuring circuit for measuring streaming potentials generated between the plurality of pairs of electrodes provided for respective films or spots.

8. The apparatus according to claim 1, comprising:

a pair of pressure detectors which are disposed respectively at the inlet and the outlet; and a calculating circuit for calculating zeta potential based upon both the measured streaming potential and the detected pressure.

9. A method for detecting interactions between biomolecules and ligands using a reaction block constituting a reaction system comprising a reaction region including immobilized biomolecules, a supply flow channel connected to the reaction region for supplying a sample solution, and a recovery flow channel connected to the reaction region for recovering the sample solution which passes through at least a part of the reaction region,
the method comprising the step of measuring a streaming potential of the immobilized biomolecules while supplying the sample solution to the reaction region from the supply channel and recovering the sample solution passing through at least a part of the reaction region from the recovery flow channel.

10. The method according to claim 9, the method comprising the steps of
detecting pressure of the sample solution at an inlet on a side of the supply flow channel and an outlet on a side of the recovery flow channel, respectively; and
calculating a zeta potential based upon both the measured streaming potential and the detected pressure.

# FIG. 1

EP 1 371 974 A1

# FIG. 2

FIG. 3

FIG. 4

FIG. 5

FIG. 6

EP 1 371 974 A1

# FIG. 7

FIG. 8

EP 1 371 974 A1

# FIG. 9

# FIG. 10

# FIG. 11

# EP 1 371 974 A1

<table>
<tr><td colspan="2"><strong>INTERNATIONAL SEARCH REPORT</strong></td><td>International application No.<br><br>PCT/JP02/01269</td></tr>
</table>

| A. CLASSIFICATION OF SUBJECT MATTER |
|---|
| Int.Cl$^7$  G01N27/26, G01N27/327, G01N27/416, G01N33/53 |
| According to International Patent Classification (IPC) or to both national classification and IPC |

| B. FIELDS SEARCHED |
|---|
| Minimum documentation searched (classification system followed by classification symbols)<br>    Int.Cl$^7$  G01N27/26, G01N27/327, G01N27/416, G01N33/53, G01N37/00 |

Documentation searched other than minimum documentation to the extent that such documents are included in the fields searched

| Jitsuyo Shinan Koho | 1922–1996 | Toroku Jitsuyo Shinan Koho | 1994–2002 |
| Kokai Jitsuyo Shinan Koho | 1971–2002 | Jitsuyo Shinan Toroku Koho | 1996–2002 |

Electronic data base consulted during the international search (name of data base and, where practicable, search terms used)
    JICST FILE(JOIS)

| C. DOCUMENTS CONSIDERED TO BE RELEVANT |
|---|

| Category* | Citation of document, with indication, where appropriate, of the relevant passages | Relevant to claim No. |
|---|---|---|
| X<br>Y | JP, 2-257052, A  (NGK Spark Plug Co., Ltd.),<br>17 October, 1990 (17.10.90),<br>Full text; Figs. 1 to 4<br>Full text; Figs. 1 to 4<br>(Family: none) | 1,5,9<br>2,3,4,6,7,8,10 |
| Y | JP, 7-239313, A  (Shimadzu Corp.),<br>12 September, 1995 (12.09.95),<br>Full text; Figs. 1 to 3<br>(Family: none) | 2,8,10 |
| Y | JP, 4-50758, A  (Ebara Infiruko K.K., Ebara Research<br>Co., Ltd.),<br>19 February, 1992 (19.02.92),<br>Full text; Figs. 1 to 11<br>(Family: none) | 2,8,10 |
| Y | Sabine Koch et al., Protein Detection with a novel<br>ISFET-based zata potential analyzer. Biosensors &<br>Bioelectronics, 1999, Vol.14, pages 413 to 421 | 6 |

| ☒ Further documents are listed in the continuation of Box C. | ☐ See patent family annex. |
|---|---|

| * Special categories of cited documents: |
|---|
| "A" document defining the general state of the art which is not considered to be of particular relevance |
| "E" earlier document but published on or after the international filing date |
| "L" document which may throw doubts on priority claim(s) or which is cited to establish the publication date of another citation or other special reason (as specified) |
| "O" document referring to an oral disclosure, use, exhibition or other means |
| "P" document published prior to the international filing date but later than the priority date claimed |
| "T" later document published after the international filing date or priority date and not in conflict with the application but cited to understand the principle or theory underlying the invention |
| "X" document of particular relevance; the claimed invention cannot be considered novel or cannot be considered to involve an inventive step when the document is taken alone |
| "Y" document of particular relevance; the claimed invention cannot be considered to involve an inventive step when the document is combined with one or more other such documents, such combination being obvious to a person skilled in the art |
| "&" document member of the same patent family |

| Date of the actual completion of the international search<br>    04 March, 2002 (04.03.02) | Date of mailing of the international search report<br>    09 April, 2002 (09.04.02) |
|---|---|
| Name and mailing address of the ISA/<br>    Japanese Patent Office | Authorized officer |
| Facsimile No. | Telephone No. |

Form PCT/ISA/210 (second sheet) (July 1998)

20

**INTERNATIONAL SEARCH REPORT**

| International application No. |
| --- |
| PCT/JP02/01269 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y | Victor N. Morizof et al., Electrospray Deposition as a Method To Fabricate Functionally Active Protein Films. Anal. Chem., 1999, Vol.71, No.7, pages 1415 to 1420 | 2,3,7 |
| Y | Victor N. Morizof et al., Electrospray Deposition as a Method for Mass Fabrication of Mono-and Multicomponent Microarrays of Biological and Biologically Active Substances. Anal. Chem., 1999, Vol.71, No.15, pages 3110 to 3117 | 2,3,7 |
| A | JP, 6-265551, A  (Hoechst Japan Ltd.), 22 September, 1994 (22.09.94), Full text; Fig. 1 (Family: none) | 1-10 |
| A | JP, 10-332621, A  (Shimadzu Corp.), 18 December, 1998 (18.12.98), Full text; Figs. 1 to 7 (Family: none) | 1-10 |
| A | JP, 10-104188, A  (NEC Corp.), 24 April, 1998 (24.04.98), Full text; Fig. 1 (Family: none) | 1-10 |
| A | JP, 9-292358, A  (Horiba, Ltd.), 11 November, 1997 (11.11.97), Full text; Figs. 1 to 3 (Family: none) | 1-10 |

Form PCT/ISA/210 (continuation of second sheet) (July 1998)